(19) **Europäisches Patentamt**

**European Patent Office**

**Office européen des brevets**

(11) Veröffentlichungsnummer: **0 277 528**
**B1**

(12) # EUROPÄISCHE PATENTSCHRIFT

(45) Veröffentlichungstag der Patentschrift:
27.12.90

(51) Int. Cl.⁵: **A61F 2/24**

(21) Anmeldenummer: **88100557.3**

(22) Anmeldetag: **16.01.88**

(54) **Herzklappenprothese.**

(30) Priorität: **22.01.87 DE 3701702**

(43) Veröffentlichungstag der Anmeldung:
**10.08.88 Patentblatt 88/32**

(45) Bekanntmachung des Hinweises auf die Patenterteilung:
**27.12.90 Patentblatt 90/52**

(84) Benannte Vertragsstaaten:
**AT BE CH DE ES FR GB GR IT LI LU NL SE**

(56) Entgegenhaltungen:
**EP-A- 0 070 259**
**WO-A-85/04094**
**DE-A- 2 753 159**

**ENGINEERING IN MEDICINE, Band 16, Nr. 2, April 1987,**
**Seiten 67-76, MEP Ltd, London, GB; H. REUL et al.:**
**"Laboratory testing of prosthetic heart valves"**

(73) Patentinhaber: **B. Braun Melsungen AG, Carl-Braun**
**Strasse, D-3508 Melsungen(DE)**

(72) Erfinder: **Knoch, Martin, Dr. Ing., Kullenhofwinkel 34,**
**D-5100 Aachen(DE)**
Erfinder: **Reul, Helmut, Prof. Dr. Ing., Akazienstrasse 65,**
**D-5160 Düren(DE)**
Erfinder: **Rau, Günter, Prof. Dr. rer. nat., Fuchserde 50,**
**D-5100 Aachen(DE)**

(74) Vertreter: **Selting, Günther, Dipl.-Ing. et al,**
**Patentanwälte von Kreisler, Selting, Werner**
**Deichmannhaus am Hauptbahnhof, D-5000 Köln 1(DE)**

## Beschreibung

Die Erfindung betrifft eine Herzklappenprothese nach dem Oberbegriff des Patentanspruchs 1.

Die natürlichen Herzklappen sind drei- bzw. zweiflügelige Segelklappen, die technisch gesprochen die Funktion von Rückschlagventilen haben, welche das Blut in einer Richtung durchlassen, in Gegenrichtung jedoch sperren. Beim Ersatz der natürlichen Herzklappen durch mechanische Pendel- oder Kippscheibenprothesen werden einflügelige oder zweiflügelige Klappen eingesetzt, bei denen klappenförmige Schließkörper in einem Klappenring, der an der betreffenden Öffnung des Herzens festgenäht wird, durch den Blutdruck bzw. die Blutströmung bewegbar sind. Beim Langzeiteinsatz solcher Herzklappenprothesen können sich schwerwiegende Probleme ergeben, die eine lebenslange Einnahme von Antikoagulantien durch den Patienten oder das Auswechseln der Prothese erforderlich machen.

Bei den bekannten einflügeligen Herzklappenprothesen besteht der Schließkörper aus einer ebenen oder gewölbten Klappe oder aus einem Profilkörper, der nach Art eines Flugzeugflügels geformt ist. Bei einer Herzklappenprothese, von der der Oberbegriff des Patentanspruchs 1 ausgeht (DE-OS 27 53 159) ist der Schließkörper derart gewölbt, daß seine quer zur Schwenkachse verlaufende Skelettlinie in Hauptströmungsrichtung durchgebogen ist. Durch die Wölbung soll eine Vergrößerung des Öffnungswinkels erreicht werden.

Ein gemeinsamer Nachteil einflügeliger Klappenprothesen besteht darin, daß die Strömung auch in voll geöffneter Stellung zur Erzeugung eines ausreichenden Öffnungsmomentes einseitig abgelenkt werden muß. Das Öffnungsmoment, mit dem der Schließkörper gegen seinen Anschlag gedrückt wird, resultiert im wesentlichen aus der Richtungsänderung der Strömung. Voraussetzung für einen großen Öffnungswinkel ist demnach eine starke Strömungsablenkung durch den Schließkörper. Die dabei entstehende Strömungsform ist im Fall des Aortenklappenersatzes unphysiologisch. Die Folge sind vergleichsweise hohe Druckverluste und ein ausgedehntes Totwassergebiet in der Aorta ascendens. Die Strömung wird stark verwirbelt und ist auch in der Hauptströmungsphase starken Fluktuationen unterworfen, da die von den Strahlrändern ausgehenden Wirbelstrukturen ungeordnet abschwimmen. Im oberen Bereich der Aortenbulben bildet sich einseitig ein ausgeprägter Staupunkt, der die Aortenwand belastet.

Der Erfindung liegt die Aufgabe zugrunde, eine Herzklappenprothese der im Oberbegriff des Patentanspruchs 1 angegebenen Art derart weiterzubilden, daß in geöffneter Stellung ein ausreichend großes Öffnungsmoment wirksam ist, ohne daß eine zu starke Ablenkung der Blutströmung in Richtung auf die Aortenwand erfolgt.

Die Lösung dieser Aufgabe erfolgt erfindungsgemäß mit den im kennzeichnenden Teil des Patentanspruchs 1 angegebenen Merkmalen.

Bei der erfindungsgemäßen Herzklappenprothese ist die Skelettlinie, d.h. die Profilmittellinie in der quer zur Schwenkachse verlaufenden Mittelebene des Schließkörpers, S-förmig gekrümmt. Dadurch wird erreicht, daß die Strömung im vorgegebenen Öffnungswinkel parallel zur Anströmkante des Schließkörpers verläuft, während die Umlenkung der Strömung im Bereich des Schließkörperzentrums, also stromab von der exzentrisch angeordneten Schwenkachse, erfolgt, so daß das Öffnungsmoment ausschließlich im Zentralbereich des Schließkörpers erzeugt wird. Die Abströmkante ist ebenfalls parallel zur Hauptströmungsrichtung gerichtet. Die S-förmige Geometrie begünstigt die Bildung eines stabilen geschlossenen Wirbels auf der Saugseite des Schließkörpers, mit der Folge einer Verkleinerung des Totwassergebietes in der Aorta ascendens. Da die Strömung weniger stark abgelenkt wird als bei konvex-konkaver Schließkörpergeometrie und anschließend wieder in paralleler Richtung zur Aortenachse geführt wird, kann die Schwenkbewegung zum Öffnen und Schließen bei vergleichbarem Druckverlust entscheidend reduziert werden. Während normalerweise der Öffnungswinkel 80° beträgt, liegt er bei der erfindungsgemäßen Herzklappenprothese in der Größenordnung von 72°. Weiterhin wird durch die günstigere Strömungsführung am Schließkörper der Staudruck an der Aortenwand abgeschwächt.

Der während der Hauptströmungsphase vorliegende stabile Wirbel vergrößert sich bei der Druckumkehr gegen Systolenende und unterstützt wesentlich den Schließvorgang. Das Schließgeräusch wird durch die dämpfende Wirkung des Wirbels vermindert. Nach Klappenschluß werden die Aortenbulben während der Diastole großräumig ausgespült. Infolge der Verringerung des Totwassergebietes wird die Gefahr der Thrombenbildung verringert, so daß das Erfordernis einer lebenslangen Einnahme von Antikoagulantien durch den Patienten wesentlich abgeschwächt wird.

Bei einer zusätzlichen Wölbung des Schließkörpers quer zur Skelettlinie wird die seitliche Randumströmung des Schließkörpers begünstigt. In geöffneter Stellung ist der Schließkörper aus der Schwenkachse heraus zur Ringachse hin gewölbt. Dadurch wird der stromab von der Schwenkachse gelegene Wirbel auf der Saugseite verstärkt und das Totwasser in der Aorta ascendens durch zwei gegenläufige, sich in Strömungsrichtung aufweitende Wirbelzöpfe ausgewaschen. Zwar sind die Druckverluste bei dieser Ausführungsform durch die Dissipation der Sekundärströmung geringfügig erhöht, jedoch wird dies durch den Vorteil der großräumigen Wirbelstrukturen ausgeglichen.

Vorteilhafte Weiterbildungen und Ausgestaltungen der Erfindung sind in den Unteransprüchen angegeben.

Im folgenden werden unter Bezugnahme auf die Zeichnungen Ausführungsbeispiele der Erfindung näher erläutert.

Es zeigen:

Fig. 1 einen Querschnitt durch eine implantierte Herzklappenprothese,

Fig. 2 einen Schnitt entlang der Linie II-II von Fig. 1,

Fig. 3 eine separate Darstellung des Schließkörpers zur Erläuterung der Schließkörpergeometrie,

Fig. 4 ein zweites Ausführungsbeispiel der Herzklappenprothese im implantierten Zustand,

Fig. 5 einen Schnitt entlang der Linie V-V von Fig. 4, und

Fig. 6 die Wirbelstruktur des Strömungsverlaufs bei der Herzklappenprothese nach Fign. 4 und 5.

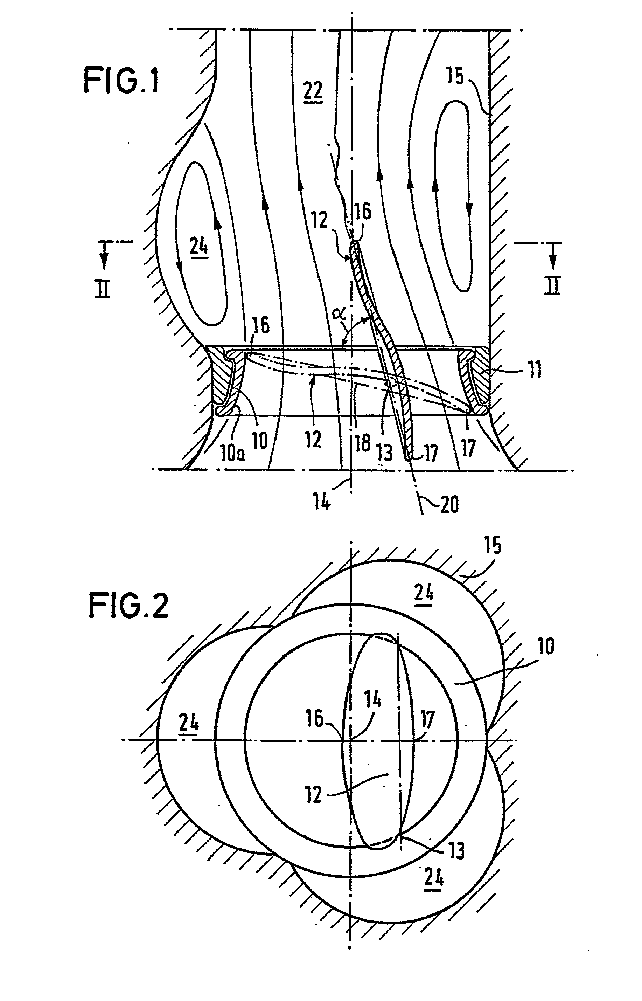

Die Herzklappenprothese nach den Fign. 1 bis 3 weist einen kreisförmigen Klappenring 10 auf, dessen rotationssymmetrische Innenfläche 10a sich in Strömungsrichtung stetig zunehmend verengt. Der Klappenring 10 ist von einem Nahtring 11 umgeben, der an das Aortengewebe 15 angenäht werden kann. Im Klappenring 10 ist der Schließkörper 12 schwenkbar gelagert. Die Schwenkachse 13 ist exzentrisch angeordnet, d.h. im Abstand von der Ringachse 14.

Der Schließkörper 12 besteht aus einem starren Flächenmaterial, dessen Wandstärke vorzugsweise an allen Stellen gleich ist. Im Schließzustand liegt die äußere Kante 16 des großen Flügelabschnitts von innen her am Auslaßbereich der Innenfläche 10a, während sich die äußere Kante 17 des kleineren Flügelabschnitts gegen den Einlaufbereich der Innenfläche 10a legt. Im Schließzustand liegt der Schließkörper 12 also diagonal im Innern des Klappenringes, so wie dies in Fig. 1 dargestellt ist. Im Öffnungszustand verläuft die Schließkörper-Hauptebene, in der die Kanten 16 und 17 liegen, unter einem Öffnungswinkel α zur Ringebene, der α = 65° bis 75° beträgt. Ein (nicht dargestellter) Anschlag begrenzt die Öffnungsbewegung des Schließkörpers 12 in Bezug auf den Klappenring 10. Die Schwenkachse 13 ist keine physische Achse, sondern eine geometrische Achse, auf der zwei Lagerzapfen angeordnet sind, die nach entgegengesetzten Seiten von dem Rand des Schließkörpers 12 abstehen und in entsprechende Lagerausnehmungen des Klappenrings 10 eintauchen. Der umlaufende Rand 18 des Schließkörpers liegt in einer einzigen Ebene und ist zur Anpassung an die Geometrie der Innenfläche 10a des Klappenrings 10 im wesentlichen ellipsenförmig gestaltet. Die Kanten 16 und 17 sind Bestandteile dieses Randes 18.

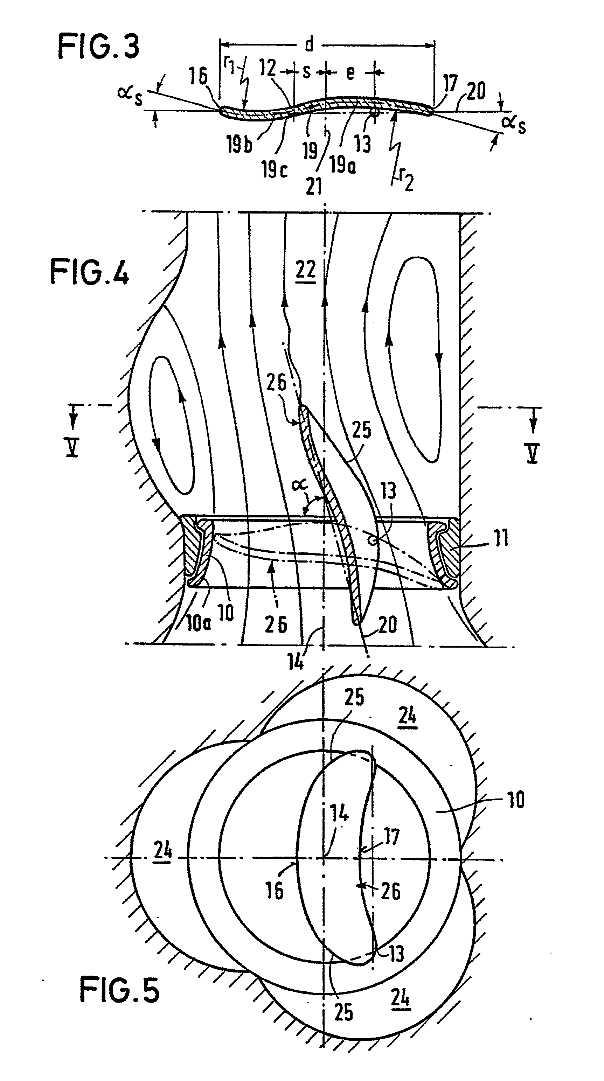

In Fig. 3 ist der Verlauf der Skelettlinie des Schließkörpers 12 dargestellt. Mit Skelettlinie ist diejenige Mittellinie des Profils bezeichnet, die in einer quer zur Schwenkachse 13 verlaufenden Ebene liegt, und zwar in der Quermittelebene des Schließkörpers. In dieser Ebene ist die S-förmige Struktur des Schließkörpers am stärksten ausgeprägt; mit zunehmenden Abstand von der Skelettlinienebene (in Richtung der Schwenkachse 13) wird die S-Struktur immer flacher, um schließlich in die Ebene des Randes 18 auszulaufen.

Der im Bereich der Schwenkachse 13 liegende Teil 19a der Skelettlinie ist (im Schließzustand des Schließkörpers) in Hauptströmungsrichtung gewölbt, während der entfernt von der Schwenkachse 13 liegende Teil 19b in Gegenrichtung gewölbt ist. Die beiden Teile 19a und 19b bilden jeweils Kreisbögen, wobei der Radius $r_2$ des Teils 19a größer ist als der Radius $r_1$ des Teils 19b. Die Teile 19a und 19b gehen im Wendepunkt 19c knickfrei ineinander über. Durch den Wendepunkt 19c verläuft auch die durch die Kanten 16 und 17 hindurchgehende Sehne 20. Der Abstand der beiden Kanten 16 und 17 der Skelettlinie, also der Durchmesser des Schließkörpers 12, ist mit d bezeichnet. Der Abstand der Schwenkachse 13 von der Mittelachse 21 des Schließkörpers ist e und der Abstand des Wendepunkts 19c von der Mittelachse 21 ist s. Der Winkel $α_S$, unter dem der Kantenbereich 17 in Bezug auf die Sehne 20 verläuft, ist gleich dem Winkel, unter dem der Kantenbereich 16 in Bezug auf die Sehne 20 verläuft. Dieser Winkel $α_S$ beträgt vorzugsweise 5° bis 15°. Ein bevorzugter Bereich für den Quotienten e : d ist 0,15 bis 0,25 und ein bevorzugter Bereich für den Quotienten s : d ist 0,1 bis 0,2.

Die Radien $r_1$ und $r_2$ der gewölbten Teile 19b und 19a sind:

$$r_1 = \frac{\frac{1}{4} - \frac{1}{2}\frac{s}{d}}{\sin α_s} \cdot d$$

$$r_2 = \frac{\frac{1}{4} + \frac{1}{2}\frac{s}{d}}{\sin α_s} \cdot d$$

In Fig. 1 ist der beschriebene Schließkörper zusätzlich in Öffnungsstellung dargestellt, wobei die sich in der Öffnungstellung des Schließkörpers ergebenden Stromlinien angegeben sind. Die Herzklappenprothese ist am Eingang der Aorta ascendens 22 angeordnet. Hinter der Herzklappe befinden sich in der Aorta ascendens drei sternförmig angeordnete Aortenbulben 24, die die Segel der natürlichen dreiflügeligen Herzklappe umgeben. Wenn die natürliche Herzklappe entfernt und durch die Herzklappenprothese ersetzt ist, beeinflussen die Aortenbulben 24 die Blutströmung, wobei Rezirkulationsgebiete bzw. Totwasserbereiche entstehen. Wie in den Fign. 1 und 2 dargestellt ist, wird die Herzklappenprothese so eingesetzt, daß der geöffnete Schließkörper 12 etwa parallel zu dem einen Aortenbulbus 24 verläuft. Wie erkennbar ist, sind die Kanten 16 und 17 im Öffnungszustand parallel zur ankommenden bzw. abgehenden Strömung ausgerichtet. Die durch die Strömung auf den Mittelbereich des Schließkörpers 12 ausgeübte Kraft drückt den Schließkörper in die Öffnungstellung und hält ihn gegen den (nicht dargestellten) Anschlag gedrückt. Bei ausreichender Öffnungskraft für einen flatterfreien Anschlag wird die Strömung durch den geöffneten Schließkörper nicht wesentlich abgelenkt. Insbesondere wird die Blutströmung nicht auf eine bestimmte Stelle der Aortenwand 15 konzentriert, so daß kein ausgeprägter Staupunkt entsteht. Der durch den Schließkörper verursachte Druckverlust ist trotz des kleineren Öffnungswinkels geringer als bei den üblichen konvex-konkaven Schließkörpern. Die Schließdauer und damit auch das Rückströmvolumen sind aufgrund des kleineren Schwenkwinkels und der größeren Angriffsfläche bei einsetzendem Rückstrom erheblich reduziert.

Bei der in den Fign. 4 und 5 dargestellten Herzklappenprothese ist der Klappenring 10 in gleicher Weise ausgebildet wie bei dem ersten Ausführungsbeispiel und auch die Skelettlinie des Schließkörpers 26 entspricht derjenigen des Schließkörpers des ersten Ausführungsbeispiels. Der Unterschied besteht darin, daß der Schließkörper 26 zusätzlich quer zur Ebene des Skelettlinienquerschnitts, die der Schnittebene von Fig. 4 entspricht, gewölbt ist. Durch diese Wölbung wird der Querschnitt der kleineren Öffnung, die im Klappenring entsteht, wenn der Schließkörper in Öffnungsstellung ist, vergrößert. Außerdem werden ungünstig spitze Winkel zwischen der Ringinnenfläche und den Schließkörperoberflächen an den Lagerstellen vermieden. Dies führt zu einer Verbesserung der Auswaschung der Aorta ascendens und zu einer zusätzlichen Verringerung der Neigung zur Thrombenbildung. Ein weiterer Vorteil dieser Ausführungsform besteht in den erhöhten Öffnungs- und Schließmomenten durch Vergrößerung des Hebelarms für die angreifenden Strömungskräfte. Die Seitenränder 25 verlaufen bei dieser Ausführungsform nicht in einer Ebene, sondern sie erstrecken sich (bei geschlossenem Schließkörper 26) in Strömungsrichtung. Die Schwenkachse 13, an der der Schließkörper in der Nähe der Seitenränder 25 gelenkig mit dem Klappenring 10 verbunden ist, befindet sich stromabwärts von der Skelettlinie des Klappenrings, während sie bei dem ersten Ausführungsbeispiel stromaufwärts von der Skelettlinie angeordnet ist. Der maximale Öffnungswinkel $\alpha$ beträgt auch bei dem zweiten Ausführungsbeispiel 65° bis 75°.

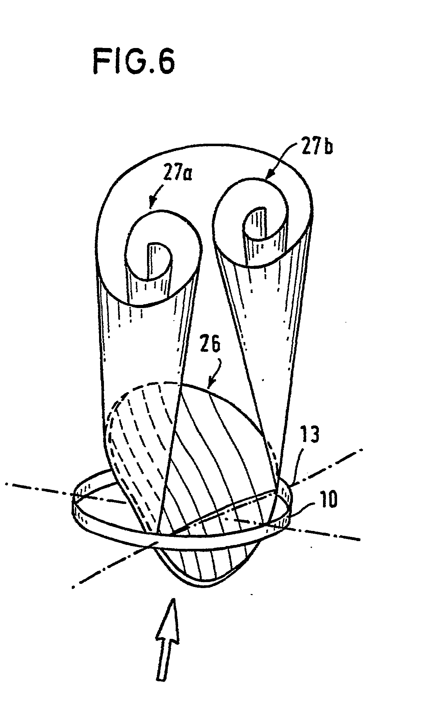

In Fig. 6 ist der Verlauf der Randumströmung des Schließkörpers 26 dargestellt. Deutlich sind die beiden Wirbelzöpfe 27a und 27b zu erkennen, in denen die Strömung um parallel zur Ringachse verlaufende Achsen rotiert und die das Totwasser in der Aorta ascendens auswaschen. Die Wirbelzöpfe 27a und 27b gehen jeweils von der Schwenkachse 13 aus und weiten sich in Strömungsrichtung auf.

Obwohl die Herzklappenprothese anhand ihrer Anwendung als Aortenklappe beschrieben wurde, kann sie in gleicher Form auch nach Modifikation des textilen Nahtringes 11 als Mitralklappe benutzt werden.

**Patentansprüche**

1. Herzklappenprothese mit einem Klappenring (10), in dem ein Schließkörper (12;26) um eine quer und mit Abstand zur Ringachse (14) verlaufende Schwenkachse (13) schwenkbar gelagert ist, wobei die in einer Normalenebene zur Schwenkachse (13) verlaufende Skelettlinie (19) des Schließkörpers (12;26) gebogen ausgebildet ist,
**dadurch gekennzeichnet,**
daß die Skelettlinie (19) S-förmigen Verlauf hat, wobei ein im Bereich der Schwenkachse (13) liegender erster Teil (19a) der Skelettlinie in geschlossener Stellung in Hauptströmungsrichtung gewölbt ist, während ein entfernt von der Schwenkachse (13) liegender zweiter Teil (19b) der Skelettlinie entgegen der Hauptströmungsrichtung gewölbt ist.

2. Herzklappenprothese nach Anspruch 1, dadurch gekennzeichnet, daß die äußeren Endbereiche (16,17) der beiden gewölbten Teile (19b,19a) mit einer die Kanten des Schließkörpers (12;26) in der Skelettlinie (19) schneidenden Sehne (20) Winkel ($\alpha_S$) von 5° bis 15° bilden.

3. Herzklappenprothese nach Anspruch 1 oder 2, dadurch gekennzeichnet, daß der Wendepunkt (19c) zwischen beiden Teilen (19a,19b) auf der Skelettlinie (19) etwa in der Mitte zwischen der Schwenkachse (13) und dem Endbereich (16) des zweiten Teils (19b) angeordnet ist.

4. Herzklappenprothese nach Anspruch 2, dadurch gekennzeichnet, daß der Wendepunkt (19c) zwischen beiden Teilen (19a,19b) wenigstens annähernd auf der Sehne (20) liegt.

5. Herzklappenprothese nach einem der Ansprüche 1 bis 4, dadurch gekennzeichnet, daß die Umfangslinie (18) des Schließkörpers (12) in einer Ebene verläuft.

6. Herzklappenprothese nach einem der Ansprüche 1 bis 4, dadurch gekennzeichnet, daß der Schließkörper (26) zusätzlich quer zu der die Skelettlinie (19) enthaltenden Normalenebene zur Schwenkachse (13) in geöffneter Stellung aus der Schwenkachse (13) heraus zur Ringachse (14) hin gewölbt ist, wobei die durch die Skelettlinie (19) getrennten Hälften symmetrisch zueinander sind.

**Claims**

1. Cardiac valve prosthesis comprising a valve ring (10) in which a closing body (12; 26) is supported to be pivotal about a swivel axis (13) extending transversely to and spaced from the ring axis (14), the design of the skeleton line (19) of the closing body (12; 26) which extends in a normal plane to the swivel axis (13) being curved, characterized in that the skeleton line (19) is S-shaped, a first portion (19a) of the skeleton line in the area of the swivel axis (13) being curved in main flow direction in the closed position while a second portion (19b) of the skeleton line away from the swivel axis (13) is curved oppositely to the main flow direction.

2. Cardiac valve prosthesis as defined in claim 1, characterized in that the external end regions (16, 17) of the two curved portions (19b, 19a) form with a chord (20) intersecting the edges of the closing body (12; 26) in the skeleton line (19) angles ($\alpha_S$) of 5° to 15°.

3. Cardiac valve prosthesis as defined in claim 1, characterized in that the turning point (19c) between both portions (19a, 19b) is on the skeleton line (19) approximately in the mid between the swivel axis (13) and the end region (16) of the second portion (19b).

4. Cardiac valve prosthesis as defined in claim 2, characterized in that the turning point (19c) is situated between both portions (19a, 19b) at least approximately on the chord (20).

5. Cardiac valve prosthesis as defined in claim 1, characterized in that the peripheral line (18) of the closing body (12) extends in one plane.

6. Cardiac valve prosthesis as defined in claim 1, characterized in that transversely to the normal plane to the swivel axis (13), the closing body (26) is curved additionally in the opened position out of the swivel axis (13) towards the ring axis (14), the halves separated by the skeleton line (19) being symmetrical with respect to each other.

## Revendications

1. Prothèse de valve cardiaque comportant un anneau de valve (10), dans lequel un corps de fermeture (12; 26) est logé à pivotement autour d'un axe de pivotement (13) au tracé transversal à l'axe d'anneau (14) et situé à distance de celui-ci, la ligne d'ossature (19) du corps de fermeture (12;26), tracée dans un plan normal à l'axe de pivotement (13) étant de forme incurvée,
caractérisé en ce que
la ligne d'ossature (19) présente un tracé en S, une première partie (19a) de la ligne d'ossature située dans la zone de l'axe de pivotement (13) est convexe, en position fermée, dans la direction du courant principal, alors qu'une deuxième partie (19b) de la ligne d'ossature située loin de l'axe de pivotement (13) est convexe en opposition à la direction du courant principal.

2. Prothèse de valve cardiaque selon la revendication 1, caractérisé en ce que les zones d'extrémités extérieures (16, 17) d'une partie convexe (19b, 19a) forment un angle ($\alpha$ s) de 5° à 15° avec une corde (20) en intersection avec les arêtes du corps de fermeture (12; 26) dans la ligne d'ossature (19).

3. Prothèse de valve cardiaque selon la revendication 1 ou 2, caractérisée en ce que le point d'inflexion (19c) entre les deux parties (19a, 19b) est disposé sur la ligne d'ossature (19) à peu près au milieu entre l'axe de pivotement (13) et la zone d'extrémité (16) de la deuxième partie (19b).

4. Prothèse de valve cardiaque selon la revendication 2, caractérisée en ce que le point d'inflexion (19c) est disposé au moins à peu près sur la corde (20) entre les deux parties (19a, 19b).

5. Prothèse de valve cardiaque selon l'une des revendications 1 à 4, caractérisée en ce que la ligne périphérique (18) du corps de fermeture (12) est située dans un plan.

6. Prothèse de valve cardiaque selon l'une des revendications 1 à 4, caractérisée en ce que le corps de fermeture (26) est en outre convexe, transversalement au plan normal à l'axe de pivotement (13) contenant la ligne d'ossature (19), en position ouverte, à partir de l'axe de pivotement (13) en direction de l'axe d'anneau (14), les moitiés séparées par la ligne d'ossature (19) étant symétrique entre elles.

FIG.1

FIG.2

**FIG.3**

**FIG.4**

**FIG.5**

# FIG.6